# EUROPEAN PATENT SPECIFICATION

(11) **EP 4 205 639 B1**
(45) Date of publication and mention of the grant of the patent: **09.07.2025**
(21) Application number: 22203621.2
(22) Date of filing: 25.10.2022
(51) Int. Cl.: A61B 5/00, A61B 5/02, A61B 5/389

(54) **DEVICE FOR DIAGNOSING ABNORMALITY BY MEASURING MINIMAL CHANGE IN MUSCLE**
VORRICHTUNG ZUR DIAGNOSE VON ANOMALIEN DURCH MESSUNG MINIMALER MUSKELVERÄNDERUNGEN
DISPOSITIF DE DIAGNOSTIC D'ANOMALIE PAR MESURE D'UN CHANGEMENT MINIMAL DE MUSCLE

(30) Priority: 30.12.2021 KR 20210192377
(43) Date of publication of application: 05.07.2023
(73) Proprietor: Korea Institute of Science and Technology, Seoul 02792 (KR); Korea University Research and Business Foundation, Seoul 02841 (KR)
(72) Inventor: LEE, Song Joo, 02792 Seoul (KR); KANG, Jong Woo, 13489 Gyeonggi-do (KR); KIM, Keun Tae, 02792 Seoul (KR); GEMECHU, Duguma Teshome, 02792 Seoul (KR); SEO, Eunyoung, 02792 Seoul (KR); LEE, Taehoon, 02792 Seoul (KR)
(74) Representative: advotec.

(56) References cited:
- WO-A1-2020/198131
- US-A1- 2005 065 426
- US-A1- 2019 150 771
- US-A1- 2020 375 464
- GRINSPAN GUSTAVO A. ET AL: "Surface wave elastography is a reliable method to correlate muscle elasticity, torque, and electromyography activity level", PHYSIOLOGICAL REPORTS, vol. 9, no. 15, 1 August 2021 (2021-08-01), US, XP093198320, ISSN: 2051-817X, Retrieved from the Internet <URL:https://onlinelibrary.wiley.com/doi/full-xml/10.14814/phy2.14955> DOI: 10.14814/phy2.14955

## Description

### BACKGROUND

### Technical field

The present disclosure relates to a device for diagnosing an abnormality by measuring a minimal change in muscle, and more particularly, to a device capable of providing vibration to a user's body part, and at the same time, calculating an abnormality of the body part based on a change in the muscle of the body part.

### [Description of Government-Sponsored Research]

This study was made with the support of a joint research project of KU Medicine-KIST TRC (Project title: Development of system for early diagnosis and monitoring of extremity deep vein thrombosis using sensor fusion technology, Project identification number: 2E3115J) under the supervision of the Korea Institute of Science and Technology and Korea University Ansan Hospital.

### Description of the Related Art

Deep vein thrombosis (DVT) is a disease that occurs due to blood clots in veins. This is a symptom in which venous blood in body parts (mainly in the lower extremities) stagnates when a person is in an immobile position for a long time or is in various situations where blood clots are likely to occur, causing blood clots to form in deep veins. It is also called 'economy class syndrome' because it is a disease that often occurs in passengers sitting in narrow seats during long-distance flights. If left untreated, deep vein thrombosis can cause limb necrosis, pulmonary embolism, and breathing difficulties, leading to death, so it is important to diagnose these symptoms early and prevent the condition from getting worse.

Conventionally, deep vein thrombosis was diagnosed mainly by examining a patient's body through ultrasound or through blood tests. Such a diagnosis method may be effective in a situation where a patient can receive periodic ultrasound or blood tests in a hospital, clinic, etc., but early diagnosis is difficult if it is caused by a long flight or driving. Preemptive methods such as mechanical prophylaxis (compression stockings, air compression devices, etc.) or pharmacological prophylaxis (administration of anticoagulants, etc.) exist, but in many cases, practical application is difficult, resulting in a large number of patients with deep vein thrombosis every year.

### Documents of Related Art

(Patent Document 1) KR Patent Laid-Open Publication No.10-2010-0049382;
WO 2020/198131 A1;
US 2005/065426 A1;
US 2020/375464 A1;
US 2019/150771 A1;
Grinspan Gustavo A. ET AL: "Surface wave elastography is a reliable method to correlate muscle elasticity, torque, and electromyography activity level", Physiological Reports, vol. 9, no. 15, 1 August 2021 (2021-08-01), XP093198320, US.

### SUMMARY OF THE INVENTION

The invention is set out in the appended set of claims.

Accordingly, an object of the present disclosure is to provide a diagnosis device capable of not only calculating an abnormality of a body part based on a change in muscle of a body part, but also diagnosing deep vein thrombosis (DVT) at an early stage.

A device for diagnosing an abnormality by measuring a minimal change in a muscle according to an embodiment includes a vibration unit that provides vibration to a body part of a user; a measuring unit that detects a minimal change in a muscle by measuring a change in elasticity of the body part according to the vibration; and a processing unit that calculates an abnormality of the body part based on the change in the elasticity of the body part, wherein the processing unit calculates the abnormality of the body party by using an algorithm that detects a degree to which a specific value of the muscle is far from a distribution chart by analyzing data distribution or an anomaly detection algorithm that detects whether there is anomaly in a variable.

According to an embodiment, the processing unit may calculate a risk of thrombosis based on the abnormality of the body part.

According to an embodiment, the device may further include a display unit that displays the risk of thrombosis.

According to an embodiment, the device may further include a control unit that controls an operation of the vibration unit.

According to an embodiment, the vibration unit may receive a control signal from the control unit and provide the vibration of a specific frequency through a vibrator attached to the body part.

According to an embodiment, the device may further include a pressure sensor positioned between the vibrator and the body part of the user to sense pressure according to the vibration, wherein the processing unit may be configured to indicate the pressure according to the vibration through the display unit.

The measuring unit includes an accelerometer that detects reflex vibration generated in the body part in response to the vibration.

The measuring unit includes an electromyograph that measures an electromyograph signal of the body part.

According to an embodiment, the processing unit may obtain an approximate function of each of measurement values of reflex vibration and electromyograph signal of the body part using a distribution of Bayesian probability value based on a change in the measurement values of the reflex vibration and EMG signal of the body part over time, and may convert each of the measurement values of the reflex vibration and EMG signal of the body part into a value within a range of 0 to 1 by using the calculated approximate function.

An anomaly detection algorithm used by the processing unit includes an isolation forest algorithm that detects anomaly by splitting anomaly data based on a tree.

According to an embodiment, the display unit may numerically indicate the risk of thrombosis through a display device.

According to an embodiment, the display unit may be configured to turn on an LED element when the risk of thrombosis is greater than or equal to a threshold.

According to an embodiment, the device may be implemented to be detachable as a wearable patch.

According to an embodiment, the processing unit may be connected to wirelessly communicate with the display unit using one or more of radio frequency (RF), Wi-Fi, cellular, Bluetooth, Bluetooth Low Energy (BLE), personal area network (PAN), short-wavelength UHF, and a combination thereof.

According to an embodiment of the present disclosure, a devise for diagnosing an abnormality of a body part at an early stage by providing vibration of a specific frequency using a vibrator attached to the user's body, and measuring the reflex vibration and EMG signal of the muscle sensed in response to the vibration.

The diagnosis device according to an embodiment determines that the occurrence of deep vein thrombosis is high when an abnormal change appears in the measurement value of reflex vibration measured by an accelerometer /or the measurement value of EMG signal measured by an electromyography, and notifies the user of abnormalities in the body parts or risk of thrombosis in real time.

The diagnosis apparatus according to an embodiment includes a vibrator attached to a body part and a sensor (accelerometer, electromyography, etc.) capable of detecting a change in the body part, so that it is easy to install and use. For example, the risk of thrombosis may be measured at regular intervals using the vibrator and sensor provided under an airplane seat. Therefore, it is possible to diagnose deep vein thrombosis earlier than the conventional ultrasound or blood test methods.

### BRIEF DESCRIPTION OF THE DRAWINGS

FIG. 1 is a block diagram illustrating a concept of a devise for diagnosing an abnormality by measuring a minimal change in a muscle according to an embodiment.
FIGS. 2A to 2D illustrate a process in which a processing unit calculates a degree to which the reflex vibration or EMG signal of a body part moves away from a data distribution by using a Bayesian algorithm according to an embodiment.
FIGS. 3A to 3C illustrate a process in which a processing unit calculates an abnormality of the reflex vibration or EMG signal of a body part using an anomaly detection algorithm according to an embodiment.
FIG. 4 illustrates a device for diagnosing an abnormality that is implemented as a wearable patch and measures an abnormality by measuring a minimal change in a muscle according to an embodiment.
FIG. 5 illustrates a process in which a processing unit calculates a risk of thrombosis and a display unit warns a user through wireless communication, according to an embodiment.

### DETAILED DESCRIPTION

The terms used in the present specification have been selected as widely used general terms as possible while considering their functions, but may vary depending on the intention or custom of those skilled in the art or the advent of new technology. In addition, in a specific case, there is a term arbitrarily selected by the applicant, and in this case, the meaning will be described in the description of the corresponding specification. Therefore, it is intended to clarify that the terms used in this specification are not simply names of terms, but should be interpreted based on the actual meaning of the terms and the contents of the entire specification.

Hereinafter, the embodiments will be described in detail with reference to the accompanying drawings and the contents described in the accompanying drawings, but the scope of the claims is not limited or restricted by the embodiments.

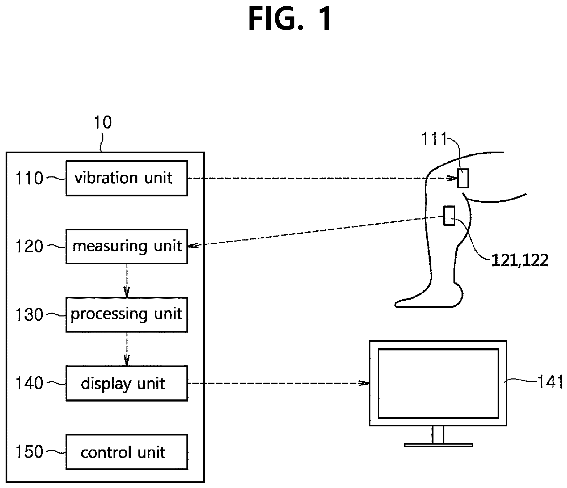

FIG. 1 is a block diagram illustrating a concept of a device for diagnosing an abnormality by measuring a minimal change in a muscle according to an embodiment. Referring to FIG. 1, a device for diagnosing an abnormality by measuring a minimal change in a muscle 10 (hereinafter, "diagnosis device") according to an embodiment is configured to include a vibration unit 110, a measuring unit 120, and a processing unit 130, a display unit 140, and a control unit 150.

Although the above components are shown separately for the convenience of conceptual description, they do not necessarily have to be implemented as independent devices or programs. For example, each of the components may be implemented by one processing unit or program, or may be implemented by an organic combination of two or more independent processing units or programs.

The vibration unit 110 receives a control signal from the control unit 150 and provides the vibration of a specific frequency through a vibrator 111 attached to a body part. The vibration unit 110 receives the control signal, for example, an input for the intensity, frequency, and form of vibration to be provided to the user (regular intensity or periodically repeating strength and weakness) from the control unit 150, and transmit it to the vibration unit 110 by wire or wirelessly.

The vibrator 111 is a device having a power device such as a motor to convert electrical energy into physical vibration, and is not limited to a specific shape or size. The vibrator 111 may be connected to the diagnosis device 10 by wire or through a wireless network (Bluetooth, WiFi, infrared communication, etc.). In addition, the vibrator 111 may be powered by a motor by wire, or may be powered by a built-in battery. In one embodiment, the vibrator 111 may be attached to the user's body part (arm, leg, etc.) through a fixing part such as a strap or adhesive tape that can be worn on the user's body part.

According to an embodiment, a pressure sensor (not shown) for sensing pressure according to vibration between the vibrator 111 and the user's body part may be further provided. The processing unit 130 to be described later may display a change in pressure according to the vibration on the display unit 140. This allows the user to know in real time whether the vibrator is providing vibration at the appropriate pressure level. Accordingly, when the pressure is too low or too high, the operations of the vibration unit 110 and vibrator 111 may be controlled through the control unit 150.

The measuring unit 120 may measure a change in elasticity of a body part according to the vibration provided by the vibration unit 110 to detect a minimal change in a muscle. For example, when deep vein thrombosis (DVT) occurs, blood clots are formed in the vein, which causes muscle stiffness, and the measuring unit 120 measures a change in elasticity of a muscle to detect these symptoms.

The measuring unit 120 detects a change in elasticity of a body part through an accelerometer 121 and an electromyography 122, and warns the user by calculating an abnormality or risk of thrombosis of the body part when an abnormal change occurs.

According to an embodiment, the measuring unit 120 may sense the reflex vibration generated in the user's body part through an accelerometer 121. Reflex vibration refers to the tremor that occurs in the surrounding muscles when an arbitrary vibrational stimulus is applied to the muscle. In general, in an environment with the same muscle quality or blood flow, when the vibration stimulation of the same characteristic (intensity, frequency, stimulation interval) is applied, the reflex vibration of the corresponding characteristic appears at a constant level. In contrast, when deep vein thrombosis occurs and the muscle becomes stiff or the blood flow changes, the characteristic of the reflex vibration sensed in the muscle changes rapidly. The accelerometer 121 is a device for converting a minimal movement of a muscle into an electrical signal, and detects a change in vibration characteristics due to the muscular dystrophy or thrombosis and transmits it to the processing unit 130. After the development of muscular dystrophy or deep vein thrombosis, the measured value of measured reflex vibration changes significantly.

According to an embodiment, the measuring unit 120 may measure an electromyograph (EMG) signal of the user's body part through an electromyograph. The electromyograph is a device that records electrical activity according to muscle contraction using the electrodes attached to or inserted into the body parts. As with the above-mentioned reflex vibration, when deep vein thrombosis occurs and the muscle is stiff or there is a change in blood flow, the EMG signal also changes. The EMG 122 detects a change in the EMG signal and transmits it to the processing unit 130. After the development of deep vein thrombosis, the measured value of measured EMG signal changes significantly.

The processing unit 130 calculates an abnormality of a body part based on a change in elasticity of the body part. The processing unit 130 may calculate an abnormality of a body part by using an algorithm that analyzes data distribution to detect a degree to which a specific value of a muscle moves away from the distribution or an anomaly detection algorithm that detects whether there is anomaly in a variable. In an embodiment, the abnormality of the body part calculated by the processing unit 130 may mean a risk of thrombosis.

The processing unit 130 combines and simultaneously uses the measurement results of reflex vibration and EMG signal (in this case, each measurement result may be given a weight), or uses each result independently to calculate an abnormality of muscle or a risk of thrombosis (the latter does not fall under the scope of the claims).

The display unit 140 displays the risk of thrombosis calculated by the processing unit 130 to the user through an external device. According to an embodiment, the display unit 140 may display the calculation result as a numerical value on a display device 141 (refer to FIG. 1). According to another embodiment, the display unit 140 may warn the user by turning on an LED element (not shown) when the abnormality of muscle or the risk of thrombosis is greater than or equal to a threshold (e.g., if the risk of deep vein thrombosis is high, a red light is turned on, and if the risk is low, a green light is turned on).

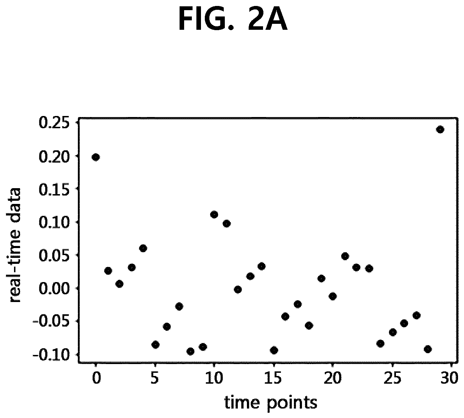

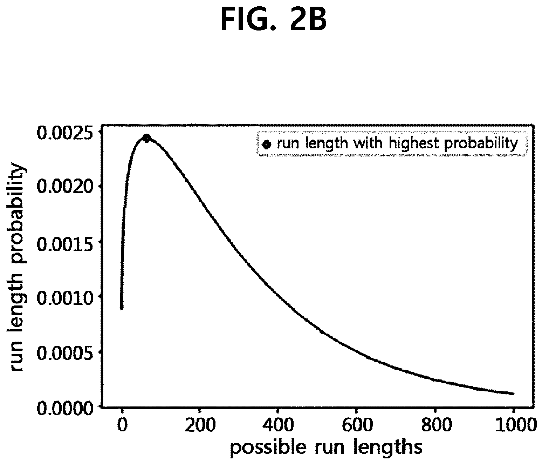

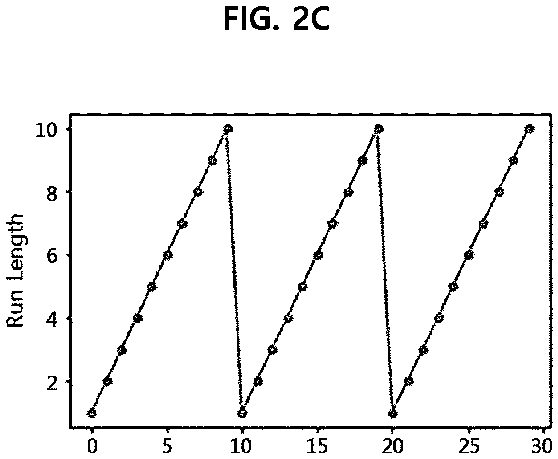

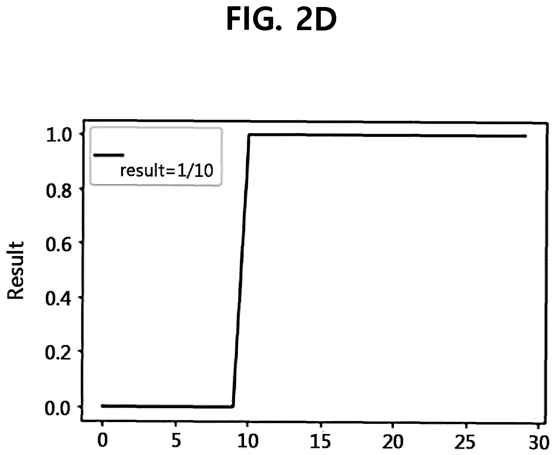

FIGS. 2A to 2D, which do not fall under the scope of the claims, illustrate a process in which the processing unit calculates a degree to which the reflex vibration or EMG signal of a body part moves away from data distribution by using a Bayesian algorithm according to an embodiment.

Referring to FIGS. 2A to 2D, the processing unit 130 may calculate the abnormality of the body part using an algorithm that detects the degree to which a specific value of muscle has moved away from the distribution chart by analyzing the data distribution of the measurement result of the reflex vibration of the body part and the measurement result of the EMG signal. The algorithm may follow the distribution of Bayesian probability values. The processing unit 130 obtains an approximate function for each of the measurement values of the reflex vibration and the EMG signal of the body part based on a change in the measurement values of the reflex vibration and EMG signal of the body part over time, and converts each of the measurement values of the reflex vibration and EMG signal of the body part into a value within a range of 0 to 1 using the obtained approximate function. Here, the measurement value of reflex vibration or EMG signal of the body part converted to a value within the range of 0 to 1 may be a value indicating the probability that the thrombosis will be measured when a muscular dystrophy or thrombosis occurs in a patient, but the embodiment is not limited thereto.

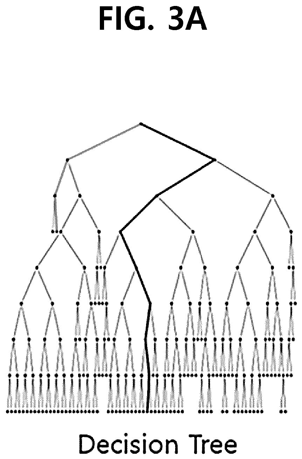

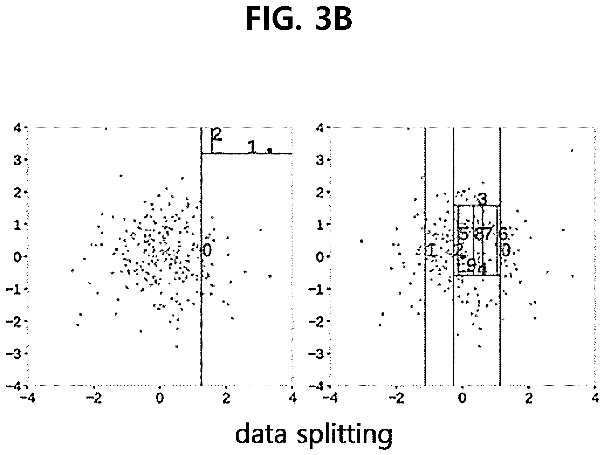

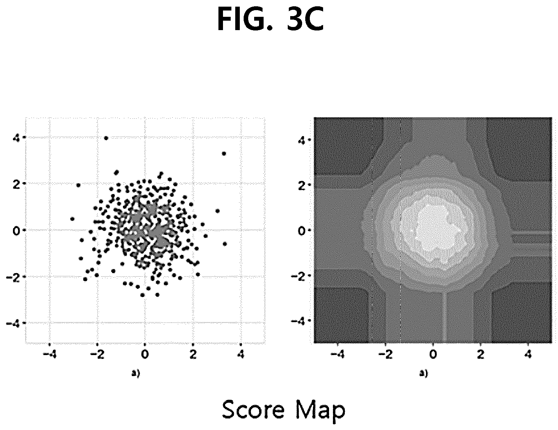

FIGS. 3A to 3C illustrate a process in which the processing unit 130 calculates an abnormality of reflex vibration or EMG signal of a body part using an anomaly detection algorithm according to an embodiment.

Referring to FIGS. 3A to 3C, the processing unit 130 can calculate an abnormality in reflex vibrations or EMG signals of a body part by using an isolation forest algorithm that detects an anomaly by isolating anomaly data based on a tree. The anomaly detection refers to detecting data that is significantly different from the majority of data or unique data. Here, anomaly may be expressed as noise, deviation, or exception. The anomaly detection is to find outlier, which is data showing different pattern in the collected data. The isolation forest technique expresses the data set in the form of a decision tree, and follows the depth direction of the decision tree in the case of splitting a normal value and uses the characteristic of splitting at the top of the decision tree in the case of splitting outlier. Using these characteristics, the isolation forest makes it possible to split normal values and outliers based on how many times the decision tree is descended and split. If there is a sudden change in the frequency component of the reflex vibration measured by the accelerometer and the frequency component of the EMG signal measured by the EMG, it is split as outlier. The greater the number of splitting, the higher the possibility of muscular dystrophy or deep vein thrombosis.

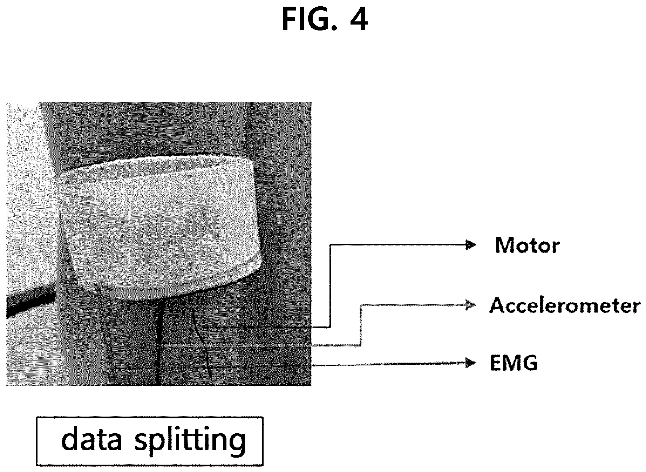

FIG. 4 illustrates a device for diagnosing abnormality that is implemented to be detachable as a wearable patch and measures a minimal change in a muscle, according to an embodiment.

Referring to FIG. 4, many wearable devices using IoT technology have recently appeared. Due to the characteristics of the wearable device, it should be easy to carry, and it should be light and capable of operating for a long time. A small device such as a wearable device is used in a form that is in direct contact with a person's body or is attached to clothes or other accessories without direct contact with the body.

In particular, among wearable devices, a wearable multi-biosignal measuring device such as a wearable electromyography device is a device that uses a sensor such as a patch-type electrode to form a contact point with various body parts (chest, wrist, ankle, etc.) of a subject to measure biosignals such as electromyography. This device is used to predict or diagnose an abnormality in body parts or the occurrence of diseases such as thrombosis by monitoring biosignals.

By implementing the diagnostic device of the present invention in a detachable manner as a wearable patch, it is possible to easily monitor bio-signals such as electromyography in daily life. Thus, medical personnel can be continuously provided with the patient's condition through the motor unit, measuring unit, and processing unit mounted on the wearable patch, and can rapidly diagnose and treat predicted disease, thereby reducing the risk of death.

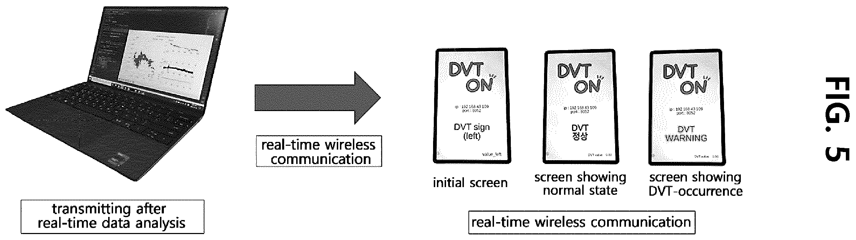

FIG. 5 illustrates a process in which the processing unit 130 calculates an abnormality of muscle or a risk of thrombosis, and a display unit warns a user through wireless communication, according to an embodiment.

The processing unit 130 may be connected to communicate wirelessly with the display unit 140 using one or more of radio frequency (RF), Wi-Fi, cellular, Bluetooth, Bluetooth Low Energy (BLE), personal area network (PAN), short-wavelength UHF, and a combination thereof.

Referring to FIG. 5, the display unit 140 displays the risk of thrombosis calculated by the processing unit 130 to the user through an external device. According to an embodiment, the display unit 140 may display the calculation result as a numerical value on the display device 141. According to another embodiment, the display unit 140 may warn the user by turning on an LED element (not shown) when the risk of thrombosis is greater than or equal to a threshold (e.g., if the risk of deep vein thrombosis is high, a red light is turned on, and if the risk of deep vein thrombosis is low, a green light is turned on).

According to the diagnostic device described above, the vibration of a specific frequency is provided using the vibrator attached to the user's body, and a muscular dystrophy or deep vein thrombosis is diagnosed by measuring the reflex vibration and EMG signal of the muscle sensed in response to the vibration. The diagnosis device according to an embodiment is configured to include the vibrator attached to a body part and the sensor capable of sensing a change in the body part, so that it is easy to install and use. For example, by using the vibrator and sensor provided under an airplane seat, it is possible to automatically measure and warn an abnormality of the body part or a risk of thrombosis of a traveler. According to this, it is possible to diagnose muscular dystrophy and deep vein thrombosis at an earlier stage compared to the conventional ultrasound examination method or blood test method.

## Claims

1. A device (10) for diagnosing an abnormality by measuring change in a muscle, comprising:
a vibration unit (110) configured to provide vibration to a body part of a user;
a measuring unit (120) configured to detect change in a muscle by measuring a change in elasticity of the body part according to the vibration; and
a processing unit (130) configured to calculate an abnormality of the body part based on the change in the elasticity of the body part,
wherein the measuring unit (120) includes an electromyography (EMG) (122) unit that is configured to measure an electromyograph signal of the body part,
wherein the processing unit (130) is configured to calculate the abnormality of the body part by using an anomaly detection algorithm that detects whether there is anomaly in a variable,
wherein the measuring unit (120) includes an accelerometer (121) that detects reflex vibration generated in the body part in response to the vibration,
**characterized in that**
the anomaly detection algorithm used by the processing unit includes an isolation forest algorithm that detects anomaly by splitting anomaly data based on a tree, wherein if there is a sudden change in the frequency component of the reflex vibration measured by the accelerometer and of the frequency component of the EMG signal measured by the EMG, it is split as outlier.

2. The device according to claim 1, wherein the abnormality of the body part calculated by the processing unit (130) is a risk of thrombosis.

3. The device according to claim 1 or 2, further comprising a display unit (140) configured to display the abnormality of the body part.

4. The device according to one of claims 1 to 3, further comprising a control unit (150) configured to control an operation of the vibration unit.

5. The device according to claim 4, wherein the vibration unit (110) receives a control signal from the control unit (150) and provides the vibration of a specific frequency through a vibrator (111) attached to the body part.

6. The device according to claim 5, further comprising a pressure sensor positioned between the vibrator and the body part of the user to sense pressure according to the vibration,
wherein the processing unit is configured to indicate the pressure according to the vibration through the display unit (140).

7. The device according to claim 3 or one of the claims 4 to 6 in connection with claim 3, wherein the display unit (140) displays a risk of thrombosis calculated by the processing unit 130 to the user.

8. The device according to claim 3 or 7 or one of the claims 4 to 6 in connection with claim 3, wherein the display unit is configured to turn on an LED element when the abnormality of the body part is greater than or equal to a threshold.

9. The device according to one of claims 1 to 8, wherein the device is detachable as a wearable patch.

10. The device according to claim 3 or 7 or 8 or one of the claims 4 to 6 and 9 in connection with claim 3, wherein the processing unit (130) is connected to wirelessly communicate with the display unit (140) using one or more of radio frequency (RF), Wi-Fi, cellular, Bluetooth, Bluetooth Low Energy (BLE), personal area network (PAN), short-wavelength UHF, and a combination thereof.

## Patentansprüche

1. Vorrichtung (10) zum Diagnostizieren einer Unregelmäßigkeit durch das Messen einer Veränderung in einem Muskel, Folgendes umfassend:
eine Vibrationseinheit (110), die dazu konfiguriert ist, einem Körperteil eines Benutzers Vibration bereitzustellen;
eine Messeinheit (120), die dazu konfiguriert ist, durch das Messen einer Veränderung einer Elastizität des Körperteils in Abhängigkeit von der Vibration eine Veränderung in einem Muskel zu erfassen; und
eine Verarbeitungseinheit (130), die dazu konfiguriert ist, eine Unregelmäßigkeit des Körperteils basierend auf der Veränderung der Elastizität des Körperteils zu berechnen,
wobei die Messeinheit (120) eine Elektromyografie-Einheit (EMG-Einheit) (122) umfasst, die dazu konfiguriert ist, ein Elektromyografie-Signal des Körperteils zu messen,
wobei die Verarbeitungseinheit (130) dazu konfiguriert ist, die Unregelmäßigkeit des Körperteils unter Verwendung eines Anamoliedetektionsalgorithmus zu berechnen, der erfasst, ob eine Anomalie einer Variable vorliegt,
wobei die Messeinheit (120) einen Beschleunigungsmesser (121) umfasst, der als Reaktion auf die Vibration eine in dem Körperteil erzeugte Reflexvibration erfasst,
**dadurch gekennzeichnet, dass**
der von der Verarbeitungseinheit verwendete Anamoliedetektionsalgorithmus einen Isolation-Forest-Algorithmus umfasst, der eine Anomalie erfasst, indem er Anomaliedaten auf der Basis eines Baums aufteilt, wobei es als ein Ausreißer aufgeteilt wird, wenn eine plötzliche Veränderung der Frequenzkomponente der Reflexvibration, die von dem Beschleunigungsmesser gemessen wird, und der Frequenzkomponente des EMG-Signals, das von der EMG gemessen wird, vorliegt.

2. Vorrichtung nach Anspruch 1, wobei die Unregelmäßigkeit des Körperteils, die von der Verarbeitungseinheit (130) berechnet wird, ein Thromboserisiko ist.

3. Vorrichtung nach Anspruch 1 oder 2, des Weiteren umfassend eine Anzeigeeinheit (140), die dazu konfiguriert ist, die Unregelmäßigkeit des Körperteils anzuzeigen.

4. Vorrichtung nach einem der Ansprüche 1 bis 3, des Weiteren umfassend eine Steuereinheit (150), die dazu konfiguriert ist, einen Betrieb der Vibrationseinheit zu steuern.

5. Vorrichtung nach Anspruch 4, wobei die Vibrationseinheit (110) ein Steuersignal von der Steuereinheit (150) empfängt und die Vibration einer bestimmten Frequenz durch einen an dem Körperteil angebrachten Vibrator (111) bereitstellt.

6. Vorrichtung nach Anspruch 5, des Weiteren umfassend einen Drucksensor, der zwischen dem Vibrator und dem Körperteil des Benutzers positioniert ist, um den Druck in Abhängigkeit von der Vibration zu fühlen,
wobei die Verarbeitungseinheit dazu konfiguriert ist, den Druck in Abhängigkeit von der Vibration durch die Anzeigeeinheit (140) anzugeben.

7. Vorrichtung nach Anspruch 3 oder einem der Ansprüche 4 bis 6 in Verbindung mit Anspruch 3, wobei die Anzeigeeinheit (140) dem Benutzer ein von der Verarbeitungseinheit (130) berechnetes Thromboserisiko anzeigt.

8. Vorrichtung nach Anspruch 3 oder 7 oder einem der Ansprüche 4 bis 6 in Verbindung mit Anspruch 3, wobei die Anzeigeeinheit dazu konfiguriert ist, ein LED-Element anzuschalten, wenn die Unregelmäßigkeit des Körperteils größer als ein oder gleich einem Schwellenwert ist.

9. Vorrichtung nach einem der Ansprüche 1 bis 8, wobei die Vorrichtung als ein tragbares Pflaster abnehmbar ist.

10. Vorrichtung nach Anspruch 3 oder 7 oder 8 oder einem der Ansprüche 4 bis 6 und 9 in Verbindung mit Anspruch 3, wobei die Verarbeitungseinheit (130) verbunden ist, um mit der Anzeigeeinheit (140) unter Verwendung von einer Hochfrequenz (HF) und/oder Wi-Fi und/oder einem Mobiltelefon und/oder Bluetooth und/oder Bluetooth Low Energy (BLE) und/oder einem Personal Area Network (PAN) und/oder einer kurzwelligen UHF und/oder einer Kombination daraus drahtlos zu kommunizieren.

## Revendications

1. Dispositif (10) pour diagnostiquer une irrégularité en mesurant un changement dans un muscle, ledit dispositif comprennent :
une unité de vibration (110) configurée pour fournir des vibrations à une partie du corps d'un utilisateur ;
une unité de mesure (120) configurée pour détecter un changement dans un muscle en mesurant un changement d'élasticité de la partie du corps en fonction de la vibration ; et
une unité de traitement (130) configurée pour calculer une irrégularité de la partie du corps sur la base du changement d'élasticité de la partie du corps,
dans lequel l'unité de mesure (120) comprend une unité d'électromyographie (EMG) (122) qui est configurée pour mesurer un signal d'électromyographie de la partie du corps,
dans lequel l'unité de traitement (130) est configurée pour calculer l'irrégularité de la partie du corps en utilisant un algorithme de détection d'anomalies qui détecte s'il y a une anomalie d'une variable,
dans lequel l'unité de mesure (120) comprend un accéléromètre (121) qui détecte une vibration de réflexe générée dans la partie du corps en réponse à la vibration,
**caractérisé en ce que**
l'algorithme de détection d'anomalies utilisé par l'unité de traitement comprend un algorithme de forêt d'isolement qui détecte une anomalie en divisant des données d'anomalie sur la base d'un arbre, dans lequel il est divisé comme donnée aberrante s'il y a un changement soudain du composant de fréquence de la vibration de réflexe mesurée par l'accéléromètre et du composant de fréquence du signal d'EMG mesuré par l'EMG.

2. Dispositif selon la revendication 1, dans lequel l'irrégularité de la partie du corps calculée par l'unité de traitement (130) est un risque de thrombose.

3. Dispositif selon la revendication 1 ou 2, comprenant en outre une unité d'affichage (140) configurée pour afficher l'irrégularité de la partie du corps.

4. Dispositif selon l'une quelconque des revendications 1 à 3, comprenant en outre une unité de contrôle (150) configurée pour contrôler un fonctionnement de l'unité de vibration.

5. Dispositif selon la revendication 4, dans lequel l'unité de vibration (110) reçoit un signal de contrôle à partir de l'unité de contrôle (150) et fournit la vibration d'une fréquence spécifique par un vibrateur (111) fixé à la partie du corps.

6. Dispositif selon la revendication 5, comprenant en outre un capteur de pression positionné entre le vibrateur et la partie du corps de l'utilisateur pour capter une pression en fonction de la vibration,
dans lequel l'unité de traitement est configurée pour indiquer la pression en fonction de la vibration par l'unité d'affichage (140).

7. Dispositif selon la revendication 3 ou l'une quelconque des revendications 4 à 6 en combinaison avec la revendication 3, dans lequel l'unité d'affichage (140) affiche un risque de thrombose calculé par l'unité de traitement (130) à l'utilisateur.

8. Dispositif selon la revendication 3 ou 7 ou l'une quelconque des revendications 4 à 6 en combinaison avec la revendication 3, dans lequel l'unité d'affichage est configurée pour allumer un élément DEL lorsque l'irrégularité de la partie du corps est supérieure ou égale à un seuil.

9. Dispositif selon l'une quelconque des revendications 1 à 8, dans lequel le dispositif est amovible comme pansement portable.

10. Dispositif selon la revendication 3 ou 7 ou 8 ou l'une quelconque des revendications 4 à 6 et 9 en combinaison avec la revendication 3, dans lequel l'unité de traitement (130) est reliée pour communiquer sans fil avec l'unité d'affichage (140) en utilisant une radiofréquence (RF) et/ou un Wi-Fi et/ou un cellulaire et/ou un Bluetooth et/ou un Bluetooth à basse énergie (BLE) et/ou un réseau personnel (PAN) et/ou une UHF à courte longueur d'onde et/ou une combinaison de ceux-ci.
